# EUROPEAN PATENT APPLICATION

(11) **EP 2 233 925 A1**
(43) Date of publication of application: **29.09.2010**
(21) Application number: 09290223.8
(22) Date of filing: 26.03.2009
(51) Int. Cl.: G01N 33/543, G01N 33/68

(54) **Immobilization method for protein having low isoelectric point**

(71) Applicant: Corning Inc., Corning, NY 14831 (US)
(72) Inventor: Van Zutphen, Steven, 77780 Bourron Marlotte (FR)
(74) Representative: Le Roux, Martine

(57) **Abstract**

A method to entrap a low isoelectric point protein, including, for example, contacting a linker-modified protein and a substrate to entrap the linker-modified protein on the substrate, the substrate surface includes a buffer solution swollen carboxy polymer surface having a pH, and the isoelectric point (pI) of the protein prior to linker modification is less than the pH of the buffer solution. Also disclosed are articles having an entrapped or immobilized protein thereon, and to methods of using the articles having an entrapped or immobilized protein, as defined herein, such as detecting or characterizing ligands specific to the protein.

## Description

The entire disclosure of any publication, patent, or patent document mentioned herein is incorporated by reference.

### BACKGROUND

The disclosure relates generally to a method for processing low isoelectric proteins for use in, for example, immobilization, isolation, characterization, analysis, diagnosis, or like applications.

### SUMMARY

The disclosure provides a method for processing a target protein having a low isoelectric point (pI) including chemically modifying the isoelectric point of the target protein to enhance the protein's interaction with or charge affinity towards a negatively biased polymer coated surface.

### BRIEF DESCRIPTION OF THE DRAWING(S)

Fig. 1 schematically shows chemical cationization of a low pI protein including its chemical modification, entrapment, and immobilization with a negatively biased surface, in embodiments of the disclosure.

Fig. 2 shows exemplary and comparative protein immobilization results, in embodiments of the disclosure.

Fig. 3 shows exemplary immobilization results for different linker to protein ratios, before and after plate washing, in embodiments of the disclosure.

Fig. 4 is a sensorgram that shows representative levels of entrapped protein, cationized and native, over time, in embodiments of the disclosure.

Figs. 5 and 6 show the amount of protein, native and cationized respectively, entrapped in a CM5 surface layer at different pH levels, in embodiments of the disclosure.

Fig. 7 shows exemplary pepsin A immobilization results with selected modified surfaces using an Epic^{®} instrument, in embodiments of the disclosure.

### DETAILED DESCRIPTION

Various embodiments of the disclosure will be described in detail with reference to drawings, if any. Reference to various embodiments does not limit the scope of the invention, which is limited only by the scope of the claims attached hereto. Additionally, any examples set forth in this specification are not limiting and merely set forth some of the many possible embodiments for the claimed invention.

In embodiments, the disclosed method provides a tool to immobilize proteins that are otherwise difficult or impossible to immobilize on, for example, a carboxylate or activated ester type three dimensional matrix. These matrices are commonly used for protein immobilization, and are examples of surfaces for use with the Epic^{®} instrument. The disclosed method for the entrapment of proteins onto the Biacore^{®} sensorchip has fewer steps than the method in Biacore's published application note 9 (*infra*) relating to low pI protein immobilization.

### Definitions

"Assay," "assaying" or like terms refers to an analysis to determine, for example, the presence, absence, quantity, extent, kinetics, dynamics, or type of a biomolecule's or a cell's optical or bioimpedance response, upon stimulation with an exogenous stimuli, such as a ligand candidate compound.

"Attached" or like term refers to any chemical interaction between two components or compounds. The type of chemical interaction that can be formed will vary depending upon the starting materials that are selected and reaction conditions. Examples of attachments described herein include, for example, covalent, electrostatic, ionic, hydrogen, or hydrophobic bonding. "Attach," "attachment," "adhere," "adhered," "adherent," "immobilized," or like terms can generally refer to immobilizing or fixing, for example, a surface modifier substance, a surface coating polymer, a compatibilizer, a cell, an antibody, a ligand candidate compound, and like entities, to a surface, such as by physical absorption, chemical bonding, and like processes, or combinations thereof. A biosensor surface can be modified, such as having a disclosed surface coating, an anchoring or tie material, a compatibilizer (e.g., fibronectin, collagen, lamin, gelatin, polylysine, etc.), or like modifications, and combinations thereof, that can promote, for example, receptivity of the biosensor surface towards particular molecular or cellular entities, such as in protein binding and ligand detection.

"Contact" or "contacting" or like terms refer to, for example, an instance of exposure by an intimate physical encounter or touching of at least one substance to another substance.

"Target" or like term refers to a protein or like substance selected for immobilization. A target can be, for example, a receptor, a phosphatase, a kinase, an enzyme, a lipoprotein, a DNA, a RNA, and like entities including whole cells having a surface protein, and synthetic or genetically engineered proteins. A receptor can be, for example, a G protein-coupled receptor (GPCR), a receptor tyrosine kinase (RTK), a transporter, an ion channel, an integrin receptor, a sodium/proton exchanger, and like entities. A kinase can be, for example, protein kinase A, protein kinase C, mitogen-activated protein (MAP) kinases, an extracellular signal-regulated kinases, Src, Rho kinase, focal adhesion kinase, and like entities. An enzyme can be, for example, a membrane-bound adenylyl cyclase, a soluble adenylyl cyclase, a protease, and like entities.

"Screen," "screening," or like terms refers to, for example, a systematic survey of one or more compounds or drug candidates or biologicals (e.g., RNAi, antibody) to examine their pharmacological activities acting on a particular target, a cell type, or a cell system. Pharmacological or biological activity is an expression describing the beneficial or adverse effects of a drug or other substance on living matter. Aspects of the disclosure are particularly useful in biosensor-based high throughput screening (HTS) applications.

"Entrap," "entrapped," "entrapping," "entrapment," and like terms refer to, for example, an association of a protein with the functionalized surface of a substrate and at sufficient level for detection with, for example, optical instrumentation, but the protein is not necessarily irreversibly attached to the surface.

"Immobilization," "immobilize," and like terms refer to, for example, an association of a protein with the surface of the substrate which is substantially irreversible, such as not susceptible to easy removal from the surface by, for example, mild repeated rinsing or soaking in buffer.

"Low pI" and like terms refers to the isoelectric point (pI) of the target protein that is less than a pH of about 5. A "low pI" target protein refers, for example, to an unmodified or native protein having an isoelectric point that is, for example, such as below about 5 to 6. The term pI, or isoelectric point, is the pH at which there is an equal number of positive and negative charges in a molecule so that the molecule carries no net charge. The pI of a protein can be determined by, for example, isoelectric focusing, or like methods.

"Protein" and like terms refers to the molecular target or like entity for entrapping, and can include, for example, a peptide, a polypeptide, a glycoprotein, a lipoprotein, a receptor, a receptor component, an antibody, and like natural or synthetic molecules, or mixtures thereof. "Protein" can include an individual protein molecule, a protein molecule in admixture, in association, in complexation, or like relations with other molecular or biological entities, including for example, whole cells, or subunits or portions thereof. Protein molecules having a pI less than or equal to about 6, and less than or equal to about 5 can be preferred in the disclosed method.

"Biosensor" or like term refers to an article, that in combination with appropriate apparatus, can detect a desired analyte. A biosensor can combine a biological component with a physicochemical detector component. A biosensor can typically consist of three parts: a biological component or element (such as tissue, microorganism, pathogen, cells, cell component, a receptor, and like entities, or combinations thereof), a detector element (operating in a physicochemical way such as optical, piezoelectric, electrochemical, thermometric, magnetic, or like manner), and a transducer associated with both components. In embodiments, the biosensor can convert a molecular recognition, molecular interaction, molecular stimulation, or like event occurring in a surface bound cell component or cell, such as a protein or receptor, into a detectable and quantifiable signal. A biosensor as used herein can include liquid handling systems which are static, dynamic, or a combination thereof. In embodiments of the disclosure, one or more biosensor can be incorporated into a micro-article. Biosensors are useful tools and some exemplary uses and configurations are disclosed, for example, in PCT Application No. PCT/US2006/013539 (Pub. No. WO 2006/108183), published Dec. 10, 2006, to Fang, Y., et al., entitled "Label-Free Biosensors and Cells," and U.S. Patent No. 7,175,980. Biosensor-based cell assays having penetration depths, detection zones, or sensing volumes have been described, see for example, Fang, Y., et al. "Resonant waveguide grating biosensor for living cell sensing," Biophys. J., 91, 1925-1940 (2006). Microfluidic articles are also useful tools and some exemplary uses, configurations, and methods of manufacture are disclosed, for example, in U.S. Patent Nos. 6,677,131, and 7,007,709. U.S. Patent Publication 20070141231 and U.S. Patent No. 7,175,980, disclose a microplate assembly and method. The compositions, articles, and methods of the disclosure are particularly well suited for biosensors based on label independent detection (LID), such as for example an Epic^{®} system or those based on surface plasmon resonance (SPR). The compositions, articles, and methods of the disclosure are also compatible with Dual Polarized Intereferometry (DPI), which is another type of LID sensor.

"EMA" generally refers to an ethylene-maleic anhydride (EMA) copolymer.

"dEMA" generally refers to "derivatized EMA," such as where residual maleic anhydride groups in the EMA copolymer are converted or derivatized with a reagent such as an alkyl amine, and like agents, or combinations thereof.

"Amine dEMA" refers to an amine derivatized EMA copolymer. A propyl amine derivatized EMA copolymer is, for example, used as a surface coating on certain Epic^{®} biosensor well-plate products commercially available from Coming, Inc.,

"Negatively biased," "negatively charged," and like terminology refers to a substrate's net charge, or a protein's net charge, and can depend on the pH of the surrounding solution. If the pH of the solution is above the pKₐ or the pI of the surface or the protein, then the surface or the protein will be negatively charged. If the pH of the solution is below the pKₐ or the pI, then the charge on surface or the protein will be either neutral or positive.

"Reactive groups" and "ionizable groups" or like terms refer to moieties that can chemically react and moieties than can ionize, respectively, and as defined in commonly owned, copending WO 2007/078873, and USSN 11/448,486. In embodiments, certain reactive groups, such as s-NHS derivatives, can be both a reactive group and an ionizable group.

A "carboxy polymer surface," "surface bound carboxylate surface," or like terms refer to a surface bound polymer containing carboxylic acid functional groups or like carboxy functional groups, such as maleic anhydride groups. The polymer of the "carboxy polymer surface," can be, for example, an ethylene-maleic anhydride (EMA) polymer according to T.Pompe (Pompe, et. al, "Functional Films of Maleic Anhydride Copolymers under Physiological Conditions," Macromol. Biosci., 2005, 5, 890-895). The polymer can be, for example, a polyacrylic acid polymer, or copolymer containing acrylic acid monomers. The polymer can be, for example, a carboxylated polysaccharide or like materials as disclosed, for example, in US Patent Nos. 5,242,828 and 5,436,161.

"Activator," "activating agent," or like terms refer to a reagent that can react with a carboxylic acid, or like surface polymer substituent or target molecule substituent, to form a reactive intermediate. The reactive intermediate has increased reactivity towards nucleophiles such as amines, thiols, alcohols, or like functional groups compared to the acid. An "activated ester" is an ester having high reactivity towards nucleophiles, for example, a maleic anhydride mer, or the reaction product of a carboxylic acid group and an activating agent. The carboxylic acid groups can be, for example, on the carboxy surface, or on a protein, or other biomolecules.

"Hydrocarbon," "hydrocarbyl," "hydrocarbylene," "hydrocarbyloxy," "oxyhydrocarbyl," and like terms refer to monovalent such as -R, or divalent such as - R- moieties, and can include, for example, alkyl hydrocarbons, aromatic or aryl hydrocarbons, alkyl substituted aryl hydrocarbons, alkoxy substituted aryl hydrocarbons, heteroalkyl hydrocarbons, heteroaromatic or heteroaryl hydrocarbons, alkyl substituted heteroaryl hydrocarbons, alkoxy substituted heteroaryl hydrocarbons, and like hydrocarbon moieties, and as illustrated herein.

"Alkyl" includes linear alkyls, branched alkyls, cycloalkyls, and like structural dispositions.

"Substituted alkyl" or "optionally substituted alkyl " refers to an alkyl substituent, which can include, for example, linear alkyls, branched alkyls, or cycloalkyls, having from 1 to 4 optional substituents selected from, for example, hydroxyl (-OH), halogen, amino (-NH₂), nitro (-NO₂), alkyl, acyl (-C(=O)R), alkylsulfonyl (-S(=O)₂R), alkoxy (-OR), and like substituents, where R is a hydrocarbyl, aryl, Het, or like moieties, such as a monovalent alkyl or divalent alkylene having from 1 to about 10 carbon atoms. For example, a hydroxy substituted alkyl, can be a 2-hydroxy substituted propylene of the formula -CH₂-CH(OH)-CH₂-, an alkoxy substituted alkyl, can be a 2-methoxy substituted ethyl of the formula -CH₂-CH₂-O-CH₃, a 1-dialkylamino substituted ethyl of the formula -CH (NR₂)-CH₃, an oligo-(oxyalkylene), poly-(oxyalkylene), or poly-(alkylene oxide) substituted alkyl, can be, for example, of the partial formula -(R-O)ₓ-, where x can be, for example, from 1 to about 50, and from 1 to about 20, and like substituted oxyalkylene substituents, such as of the formula -(CR³-CHR³-O)ₓ- where R³ is hydrogen or a substituted or unsubstituted (C₁₋₈) alkyl, and x is an integer of from 1 to about 50.

"Aryl" includes a mono- or divalent- phenyl radical or an ortho-fused bicyclic carbocyclic radical having about nine to twenty ring atoms in which at least one ring is aromatic. Aryl (Ar) can include substituted aryls, such as a phenyl radical having from 1 to 5 substituents, for example, alkyl, alkoxy, halo, and like substituents. "Het" includes a four-(4), five-(5), six-(6), or seven-(7) membered saturated or unsaturated heterocyclic ring having 1, 2, 3, or 4 heteroatoms selected from the group consisting of oxy, thio, sulfinyl, sulfonyl, and nitrogen, which ring is optionally fused to a benzene ring. Het also includes "heteroaryl," which encompasses a radical attached via a ring carbon of a monocyclic aromatic ring containing five or six ring atoms consisting of carbon and 1, 2, 3, or 4 heteroatoms each selected from the group consisting of non-peroxide oxy, thio, and N(X) wherein X is absent or is H, O, (C₁-₄)alkyl, phenyl, or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto. In embodiments, halo or halide includes fluoro, chloro, bromo, or iodo.

Alkyl, alkoxy, etc., include both straight and branched groups; but reference to an individual radical such as "propyl" embraces only the straight chain radical, a branched chain isomer such as "isopropyl" being specifically referred to.

The carbon atom content of various hydrocarbon-containing (i.e., hydrocarbyl) moieties can alternatively be indicated by a prefix designating a lower and upper number of carbon atoms in the moiety, i.e., the prefix Cᵢ₋ⱼ indicates a moiety of the integer "i" to the integer "j" carbon atoms, inclusive. Thus, for example, (C₁-C₇)alkyl or C₁₋₇alkyl refers to alkyl of one to seven carbon atoms, inclusive, and hydrocarbyloxy such as (C₁-C₈)alkoxy or C₁₋₈alkoxy refers to alkyl of one to eight carbon atoms, inclusive.

Specifically, C₁₋₇alkyl can be, for example, methyl, ethyl, propyl, isopropyl, butyl, iso-butyl, sec-butyl, tert-butyl, pentyl, 3-pentyl, hexyl, or heptyl; (C₃₋₁₂)cycloalkyl can be, for example, cyclopropyl, cyclobutyl, cyclopentyl, cyclohexyl, cycloheptyl, cyclooctyl, including bicyclic, tricyclic, or multi-cyclic substituents, and like substituents.

C₁₋₈alkoxy can be, for example, methoxy, ethoxy, propoxy, isopropoxy, butoxy, iso-butoxy, sec-butoxy, pentoxy, 3-pentoxy, hexyloxy, 1-methylhexyloxy, heptyloxy, octyloxy, and like substituents.

H-C(=O)(C₁₋₆)alkyl- or -(C₂₋₇)alkanoyl can be acetyl, propanoyl, butanoyl, pentanoyl, 4-methylpentanoyl, hexanoyl, or heptanoyl. Aryl (Ar) can be phenyl, naphthyl, anthracenyl, phenanthrenyl, fluorenyl, tetrahydronaphthyl, or indanyl. Het can be pyrrolidinyl, piperidinyl, morpholinyl, thiomorpholinyl, or heteroaryl. Heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, pyridyl, (or its N-oxide), thienyl, pyrimidinyl (or its N-oxide), indolyl, isoquinolyl (or its N-oxide), or quinolyl (or its N-oxide). A specific value for Het includes a five-(5), six-(6), or seven-(7) membered saturated or unsaturated ring containing 1, 2, 3, or 4 heteroatoms, for example, non-peroxide oxy, thio, sulfinyl, sulfonyl, and nitrogen; and a radical of an ortho-fused bicyclic heterocycle of about eight to twelve ring atoms derived therefrom, particularly a benz-derivative or one derived by fusing a propylene, trimethylene, tetramethylene or another monocyclic Het diradical thereto.

Other conditions suitable for formation and modification of the compounds, oligomers, polymers, composites, or like products of the disclosure, from a variety of starting materials or intermediates, as disclosed and illustrated herein are known. For example, see Feiser and Feiser, "Reagents for Organic Synthesis", Vol. 1, et seq., 1967; March, J. "Advanced Organic Chemistry," John Wiley & Sons, 4th ed. 1992; House, H. O., "Modem Synthetic Reactions," 2nd ed., W. A. Benjamin, New York, 1972; and Larock, R. C., "Comprehensive Organic Transformations," 2nd ed., 1999, Wiley-VCH Publishers, New York. The starting materials employed in the preparative methods described herein are, for example, commercially available, have been reported in the scientific literature, or can be prepared from readily available starting materials using procedures known in the field. It may be desirable to optionally use a protecting group during all or portions of the disclosed or alternative preparative procedures. Such protecting groups and methods for their introduction and removal are known in the art. See Greene, T. W.; Wutz, P. G. M. "Protecting Groups In Organic Synthesis," 2nd ed., 1991, New York, John Wiley & Sons, Inc.

"Include," "includes," or like terms means encompassing but not limited to, that is, inclusive and not exclusive.

"About" modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example: through typical measuring and handling procedures used for making compounds, compositions, composites, concentrates or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture. The claims appended hereto include equivalents of these "about" quantities.

"Consisting essentially of" in embodiments refers, for example, to cationized proteins, to a surface polymer composition, to a method of making or using the cationized proteins, and the surface polymer, formulation, or composition, and articles, devices, or any apparatus of the disclosure, and can include the components or steps listed in the claim, plus other components or steps that do not materially affect the basic and novel properties of the compositions, articles, apparatus, or methods of making and use of the disclosure, such as particular reactants, particular additives or ingredients, a particular agents, a particular surface modifier or condition, or like structure, material, or process variable selected. Items that may materially affect the basic properties of the components or steps of the disclosure or that may impart undesirable characteristics to aspects of the disclosure include, for example, protein denaturation, or like functional disruption or changes to the protein's molecular structure or characteristics by chemical or physical means. Thus, conditions for protein handling and processing can be selected to approximate or match conditions the protein encounters in nature including, for example, avoiding adverse pH levels and temperatures.

The indefinite article "a" or "an" and its corresponding definite article "the" as used herein means at least one, or one or more, unless specified otherwise.

Abbreviations, which are well known to one of ordinary skill in the art, may be used (e.g., "h" or "hr" for hour or hours, "g" or "gm" for gram(s), "mL" for milliliters, and "rt" for room temperature, "nm" for nanometers, and like abbreviations).

Specific and preferred values disclosed for components, ingredients, additives, reactants, reagents, polymers, oligomers, monomers, times, temperatures, and like aspects, and ranges thereof, are for illustration only; they do not exclude other defined values or other values within defined ranges. The compositions and methods of the disclosure include those having any value or any combination of the values, specific values, more specific values, and preferred values described herein.

In embodiments of the disclosure, the problem of entrapping, isolating, detecting, or other like dispositions of low pI proteins, and like substances, can be overcome by, for example, chemically modifying the charge bias or charge properties of a low pI protein or low pI protein mixture of interest. The charge bias modified protein can then be readily and selectively entrapped, isolated, detected, or other like dispositions, on a polymer coated surface, as defined herein.

During the course of our research on biosensor surface chemistries, it was discovered that the charge of a low pI protein could be chemically modified, by for example, converting carboxylate groups to, for example, organic amide groups, and surprisingly, that the chemically modified protein could then be readily entrapped, immobilized, or both, on a polymer coated substrate surface for detection, analysis, or like processing or characterization.

In embodiments, the disclosure provides compositions, articles, and methods for making and using a low pI protein.

In embodiments, advantages of present disclosure include, for example:
the method enables the entrapment and immobilization of proteins that are otherwise very difficult, if not, impossible to immobilize on a surface bound carboxylate or activated ester type three dimensional matrix. These disclosed surfaces include known matrices for protein immobilization, experimental surfaces, and a surface used in Epic^{®} biosensors. Futami, et al., have demonstrated that cationization of proteins with reagents, such as ethylene diamine, does not impair protein activity (J. Biochem., 132, 223-228 (2002)). The present disclosure uses cationization methodology to accomplish high sensitivity biochemical assays using a biosensor such as an Epic^{®} instrument or an SPR device. The disclosed immobilization methods are easier to use compared to alternative immobilization methods for low pI proteins, such as those proposed by Biacore (see: Biacore application note 9 entitled "Ligand immobilization using thioldisulphide exchange," at www.biotech.iastate.edu/facilities/protein/seminarsBIACore/ApplicationNotes/Applica tionNote9.pdf). After the chemical modification with the linker, the low pI protein becomes more reactive or attracted towards a negatively biased polymeric surface, such as found in a standard Epic^{®} biosensor surface based on activated carboxylic acids. The standard Epic^{®} biosensor surface is a robust and validated surface chemistry. Implementation of the disclosed methods can be straightforward and effective, and does not require special derivatization of the biosensor surface. Thus, commercially available or readily prepared carboxy polymer surfaces can be used, such as acrylic acid containing polymers, maleic anhydride containing polymers, and like polymer surfaces, with or without chemical activation.

In embodiments, the present disclosure provides a method where the pI of a protein is modified by reaction with a linker compound, such as an organic amine compound optionally having a second functional group. Reaction of the amine with a carboxylic acid group of the protein in the presence of a coupling agent, such as EDC, masks some or all of the negative charges that may be present on the protein. This increases the pI of the protein. The second functional group on the amine can be, for example, -CH₃, -OH, -NHR, or -NR₂ where R can be, for example, -H, methyl, ethyl, and like alkyl groups. Further addition or inclusion of basic functional groups on the amine linker will increase the pI further. Moreover, use of a primary amine, such as - NH₂, introduces an additional reactive group into the modified protein, such as -NH-, that can form covalent bonds with the matrix. In embodiments, the native low pI protein will have few or no available amine groups for reaction with the matrix. Such low pI proteins are especially difficult to immobilize using conventional methods but are readily entrapped and immobilized using the disclosed methods.

For protein binding characterization using, for example, a surface plasmon resonance (SPR) apparatus, it is desirable to attach proteins to a surface so that there is little, if any, disruption of the protein's structure and the protein's conformation. For protein binding characterization on an Epic^{®} biosensor system, excellent results have been obtained by proteins attached to a three dimensional polymer network that is anchored to a substrate surface containing carboxylate groups, and the network having been swelled in water. Protein immobilization onto such a hydrogel can occur in a two step procedure. By carrying out the immobilization at a pH lower than the pI of the protein, the first step involves the electrostatic interaction between the negatively biased or negatively charged matrix and the positively charged protein. This is referred to as the "preconcentration" step and preconcentration is generally known. The second step involves a reaction between the surface matrix and the protein to form one or more covalent bonds where the protein becomes fixed to the matrix and cannot diffuse out. If the pH is then increased, both the protein and the matrix will have a net negative charge. This induces a repulsive interaction between the protein and the matrix so that the matrix opens up, and the protein finds itself in an environment similar to that found in the cell. This is a highly desirable condition for an activity assay of an immobilized protein. Thus, in embodiments the process can further include a step of increasing the pH of the media surrounding the immobilized protein, for example prior to contacting with a ligand or like analyte substance.

A carboxy surface such as poly(ethylene-alt-maleic anhydride) derivatized with, for example, 30 to 50 mol% propylamine (i.e., dEMA) matrix used on an Epic^{®} biosensor plate has a pKₐ at around 7 and a pKₐ lower than 3. This means that in a pH range greater than 3 the polymer will carry a net negative charge. Although not limited by theory, is should be possible to immobilize any protein with a pI higher than the lowest pKₐ of the surface, in a suitable buffer with a pH above the lowest pKₐ of the surface. However, to avoid protein denaturation it is not recommended to work in a pH less than about 4.5. This means that proteins with a pI lower than about 5 are difficult to preconcentrate under standard working conditions. Although a carboxy surface swelled in a buffer of pH higher than 4.5 provides a solution for a majority of proteins, there are a number of proteins with a lower pI that cannot be immobilized using the same procedure. At the working pH there is a net negative charge on both the protein and the matrix. This means that the preconcentration step will not take place. In the methods of the disclosure, this shortcoming can be avoided by synthetically increasing the pI of these low pI proteins through chemical modification. After modification, the protein behaves like a protein having a higher pI and a standard immobilization protocol can provide satisfactory results.

The concept of cationization of biomolecules with simple amines, effectively changing the pI of a protein through chemical modification, has been noted in other uses involving low pI proteins. One example mentions that proteins can be genetically modified to contain positively charged lysine and arginine tags. These tags greatly improve the ability to covalently bind the proteins to maleic anhydride containing polymers (see Allard, et al., Bioconjugate Chemistry, 2004, 15 (3), 458-466; Allard, et al., Biotechnology and Bioengineering, 2002, 80 (3), 341-348; Allard, et al., Bioconjugate Chemistry, 2001, 12 (6), 972-979; Ladaviere, et al., Bioconjugate Chemistry, 1998, 9 (6), 655-661; Ladaviere, et al., Journal of Applied Polymer Science, 1999, 71 (6), 927-936; Ladaviere, et al., Journal of Applied Polymer Science, 1999, 72 (12), 1565-1572; Ladaviere, et al., Bioconjugate Chemistry, 2000, 11, (2), 146-152). Cationization has been used to introduce a variety of substances including proteins into cells (see Futami, et al., WO2005113793 (A1); J. G. Michael, WO 8805664 (A1); Blau, et al., Critical Reviews in Therapeutic Drug Carrier Systems, 2000, 17 (5), 425-465; Futami, et al., Journal of Bioscience and Bioengineering, 2005, 99 (2), 95-103). In these examples the cationization can be accomplished using various simple functional amines. In mass-spectroscopic analysis, molecules may need to be charged to permit or enhance detection. Introducing a positive charge into proteins for mass-spectroscopic analysis can be achieved, for example, by covalent modification, or by coordination of multivalent metal cations with the proteins (see Michel, et al., Surface and Interface Analysis, 2006, 38 (11), 1386-1392; Zhang, et al., Journal of Mass Spectrometry, 2004, 39 (7), 808-816).

For the immobilization of low pI proteins, Biacore mentions functionalizing the protein with a 2-(2-pyrdinyldithio) ethaneamine (PDEA) linker. This linker contains an amine group that reacts with the protein and an activated dithiol group; the dithiol group has very specific reactivity towards thiol (-SH) groups. Using this method, the pI of the low pI protein was increased by the linker. The functionalized protein then specifically reacts with a thiol functionalized surface. Immobilization can be carried out on a thiol-modified surface but not on the more common carboxylate or activated ester functionalized surface (see Biacore application note 9, *supra*).

In embodiments, proteins that do not immobilize under normal conditions surprisingly provided considerable immobilization levels using the presently disclosed methods. Here "normal conditions" refers to when the low pI protein has a pI above about 5 or about 6 using a surface containing carboxylic acid groups, that is, of a pKₐ lower than about 7 using a buffer having, for example, about pH 3, and about pH 4.5.

In embodiments, the substrate surface, such as polymer coated substrate surfaces, can be, for example, any negatively biased matrix having, for example, carboxylates, sulfonates, and like groups, or mixtures thereof. The polymer coated substrate surface can be, for example, a coated microwell plate biosensor, or like articles. The thickness of the polymer coat can be, for example, from about 1 to about 1,000 nm, from about 2 to about 100 nm, from about 2 to about 50 nm, from about 2 to about 25 nm, including intermediate values and ranges. The thickness of the polymer coat can be controlled by the coating method selected, such as spin, dip, spray, and like methods.

In embodiments, the disclosure provides a method to entrap a protein, including, for example:
contacting the protein, such as a low pI protein, and a mixture comprised of an activator, a linker, and a buffer, to form a linker-modified protein; and
contacting the linker-modified protein and a carboxy polymer surface to entrap the linker-modified protein on the surface, the carboxy polymer surface being swelled in a buffer having a pH, and the isoelectric point (pI) of the protein is less than the pH of the buffer.

In embodiments, the pH of the buffer used to swell the carboxy polymer coated surface can be, for example, from about 0.5 to about 6. The carboxy polymer surface can be, for example, a reaction product of a poly(alkylene-alt-maleic anhydride) copolymer and an alkyl-amine. More specifically, the carboxy polymer surface can be, for example, a poly(ethylene-alt-maleic anhydride) having from about 30 to about 50 mol% of the anhydride groups reacted with propylamine, or a like alkylamine or a substituted alkylamine. The carboxy polymer surface can be, for example, a poly(acrylic acid) or a copolymer of an acrylic acid monomer and a second monomer, such as copoly(acrylic acid-acrylamide), and a blocking agent. The blocking agents, can be, for example, 2-(2-aminoethoxy) ethanol, *N*,*N*-dimethyl ethylenediamine, ethanolamine, ethylenediamine, hydroxyl amine, methoxyethyl amine, ethyl amine, isopropyl amine, butyl amine, propyl amine, hexyl amine, 2-amino-2-methyl-1-propanol, 2-(2-aminoethyl amino) ethanol, 2-(2-aminoethoxy)ethanol, dimethylethanolamine, dibutyl ethanolamine, 1-amino-2-propanol, polyethylene glycol, polypropylene glycol, 4,7,10-trioxa-1,13-tridecanediamine, polyethylene glycol, or an amine-terminated-polyethylene glycol, Trizma^{®} hydrochloride, or any combination thereof. Many of the above mentioned blocking agents can also be selected as a linker as defined herein.

In embodiments, the linker and the protein can be, for example, in a relative mole ratio of from about 1:1 to about 50:1. In embodiments, the activator can be, for example, an activating agent comprising a reagent selected from, for example, ethyl-(N',N'-dimethylamino)propylcarbodiimide hydrochloride (EDC), EDC and N-hydroxysuccinimide (NHS), EDC and N-hydroxysulfosuccinimide (sNHS), dicyclohexylcarbodiimide (DCC), diisopropylcarbodiimide (DIC), 1-hydroxybenzotriazole (HOBt), (Benzotriazol-1-yloxy)tris(dimethylamino)phosphonium hexafluorophosphate (BOP), O-(benzotriazol-1-yl)-N,N,N',N'-tetramethyluronium hexafluorophosphate (HBTU), O-(benzotriazol-1-yl)- N,N,N',N'-tetramethyluronium tetrafluoroborate (TBTU), and like compounds, or a combination thereof. In embodiments, the linker can be, for example, an organic amine compound of the formula H₂N-R¹-Y where R¹ can be a branched or unbranched, substituted or unsubstituted (C₁₋₈) alkyl or a polyalkylene glycol, and Y can be -H, -OH, -NH₂, -NHR², -NR₂², or a combination thereof, and R² can be a branched or unbranched, substituted or unsubstituted (C₁₋₈)alkyl, or a polyethylene glycol. In specific embodiments, the linker can be, for example, an amine selected from 3-(dimethylamino)-1-propylamine (DMAPA), ethylenediamine (EDA), ethanol amine, or a combination thereof. Linker compounds having a polyalkylene glycol substituent can be, for example, of the formula H₂N-R¹-Y where R¹ is a substituted or unsubstituted polyalkylene glycol of the formula H₂N-(CH₂-CHR³-O)ₓ-Y where R³ is H, or a substituted or unsubstituted (C₁₋₈) alkyl, and x is an integer of from 1 to about 50.

Representative organic amine linker compounds and their chemical structures can be, for example:
3-(dimethylamino)-1-propylamine (DMAPA): **H₂N-CH₂-CH₂-CH₂-N(CH₃)_{2;}**
ethanol amine: **H₂N-CH₂-CH₂-OH;**
ethylene diamine: **H₂N-CH₂-CH₂-NH₂;**
2-(2-aminoethoxy)ethanol: **H₂N-CH₂-CH₂-O-CH₂-CH₂-OH;**
2,2'-oxybis(ethylamine): **H₂N-CH₂-CH₂-O-CH₂-CH₂-NH₂;**
1,8-diamino-3,6-dioxaoctane: **H₂N-CH₂-CH₂-O-CH₂-CH₂-O-CH₂-CH₂-NH₂;**
and like amines, or combinations thereof, and salts thereof.

In embodiments, a suitable buffer can be selected, for example, based upon the pH at which one desires to operate. Suitable buffers can include, for example, 20 mM sodium citrate for a pH of about 2.5 to about 5.6, 20 mM sodium acetate for a pH of about 3.7 to about 5.6, sodium phosphate for a pH of about 6.0 to about 9.0, and like buffer and pH ranges, or combinations thereof, including intermediate values and ranges. Other suitable buffers can include, for example, tris(hydroxymethyl)aminomethane (tris) for pH of about 7.0 to about 9.2, and (4-(2 hydroxyethyl)piperazine-1-ethanesulfonic acid (hepes) for a pH of about 6.8 to about 8.2. Still other buffers are disclosed in, for example, the CRC Handbook, section 8-42.

In embodiments, the protein can be present, for example, in an amount of from about 0.001 micrograms/mL to about 1,000 micrograms/mL, from about 1 micrograms/mL to about 100 micrograms/mL, and from about 10 micrograms/mL to about 100 micrograms/mL, and like amounts, including intermediate values and ranges. The linker-modified protein can be entrapped on the carboxy polymer surface, for example, in an amount having an indication of from about 10 to about 100,000 picometers, from about 100 to about 10,000 picometers, from about 200 to about 5,000 picometers, and from about 400 to about 5,000 picometers, including intermediate values and ranges, as measured by, for example, a resonance wave guide biosensor, such as a Coming, Inc., Epic^{®} instrument, and like biosensor instruments or systems.

In embodiments, the low pI protein can be, for example, at least one protein having a pI of from about 2 to about 5, including intermediate values and ranges. Suitable low pI proteins are known and include, for example, trypsin inhibitor (pI = 4.6), calmodulin (pI = 4.0), amyloglycosidase (pI = 3.6), and like proteins.

Many major classes of proteins, for example, kinases, proteases, nuclear receptors, and like proteins, may have subclasses having low isoelectric values (pIs). Table 1 lists exemplary and representative human kinases having calculated pI values less than about 5. The low pI human kinases comprise about 25 of a total of greater than about 518 of all known human kinases. The listed proteins and their sequences can be found, for example, on-line or with like resources, such as at http://kinase.com/kinbase/. The pI of a candidate or target protein can be calculated from its structure or sequence, for example, using an on-line calculator or like tools, such as "Compute pI/M_{w} tool" at http://us.expasy.org/tools/pi_tool.html.

**Table 1. Human kinases having pI values less than about 5.**

| **Kinase Name** | **MW (kDa)** | **pI** |
|---|---|---|
| LMR2_TK_Lmr | 159.57 | 4.08 |
| LMR1_TK_Lmr | 144.64 | 4.2 |
| PASK_CAMK_CAMKL_PASK | 142.88 | 4.49 |
| RSKL1_AGC_RSKL | 118.7 | 4.49 |
| SCYL3_Other_SCY1 | 76.74 | 4.58 |
| LMR3_TK_Lmr | 153.94 | 4.61 |
| SRPK2_CMGC_SRPK | 77.54 | 4.61 |
| PDGFRb_TK_PDGFR | 123.99 | 4.62 |
| Erk3_CMGC_MAPK_ERK | 82.69 | 4.67 |
| SLK_STE_STE20_SLK | 132.83 | 4.69 |
| TAF1_Atypical_TAF1 | 212.71 | 4.7 |
| MST1_STE_STE20_MST | 55.64 | 4.71 |
| DRAK1_CAMK_DAPK | 46.57 | 4.72 |
| NEK11_Other_NEK | 74.18 | 4.74 |
| NRBP1_Other_NRBP | 59.85 | 4.75 |
| PBK_Other_TOPK | 36.09 | 4.75 |
| H11_Atypical_H11 | 21.61 | 4.76 |
| SgK396_Other_Other-Unique | 115.7 | 4.79 |
| PDGFRa_TK_PDGFR | 122.68 | 4.81 |
| DMPK1_AGC_DMPK_GEK | 70.37 | 4.83 |
| MST2_STE_STE20_MST | 56.27 | 4.87 |
| SgK307_Other_NKF5 | 162.72 | 4.9 |
| DRAK2_CAMK_DAPK | 42.35 | 4.91 |
| BUBR1_Other_BUB | 119.54 | 4.92 |
| CaMK1a_CAMK_CAMK1 | 41.34 | 4.92 |

Table 2 lists exemplary and representative human proteases having calculated pI values less than or equal to about 5. The low pI human proteases comprise about 60 of a total of about 1,000 of all human proteases.

**Table 2. Human Proteases having pI values less than about 5.**

| **Protease Name** | **MW (kDa)** | **pI** |
|---|---|---|
| pepsin A | 41.98 | 3.93 |
| pepsin A4 | 42 | 3.98 |
| endogenous retrovirus retropepsin | 10.45 | 4.06 |
| ADAMTS7 peptidase | 42.44 | 4.09 |
| presenilin 2 | 50.15 | 4.23 |
| proteasome subunit alpha 5 | 26.47 | 4.4 |
| Mername-AA122 peptidase | 18.74 | 4.41 |
| secemin 1 | 46.43 | 4.42 |
| ADAM22 protein | 9.6 | 4.45 |
| cathepsin E | 42.8 | 4.46 |
| prostase | 27.03 | 4.46 |
| Memame-AA050 peptidase | 55.56 | 4.51 |

In embodiments, the method can further include forming at least one covalent bond between the substrate surface and the modified protein, for example, to provide covalent immobilization of the derivatized protein.

In embodiments, the method can further include rinsing the modified protein contacted substrate with buffer to remove, for example, unbound or free protein, such as modified or derivatized protein which is only weakly associated with the polymer coated surface and not entrapped or immobilized on the polymer coated substrate.

In embodiments, the disclosure provides a method for detecting a low pI protein including, for example:
modifying the charge on the protein by contacting the protein with a mixture of an activating agent and an amino-linker compound to form an amino-linker modified protein;
contacting a carboxy polymer surface and the amino-linker modified protein to immobilize the amino-linker modified protein on the surface; and
detecting the immobilized amino-linker modified protein,
the carboxy polymer surface comprises a buffer swollen surface having a pH from about 0.5 to about 7, and the isoelectric point (pI) of the protein being less than the pH of the substrate.

In embodiments, the detection of the immobilized the amino-linker modified protein on the surface can be accomplished, for example, with a biosensor.

In embodiments, the carboxy polymer coated surface can be, for example, a reaction product of a poly(alkylene-alt-maleic anhydride), such as being previously associated with the surface of a biosensor with, for example, and aminosilane or like conversion agents, and an alkyl-amine, on the surface of a microwell plate biosensor. Such carboxy polymer coated surfaces having a poly(alkylene-alt-maleic anhydride) or an amine derivative are commercially available from, for example, Coming, Inc, such as in the EPIC^{®} microplate.

In embodiments, the carboxy polymer coated surface can be, for example, at least one of:
an alkyl-amine modified poly(alkylene-alt-maleic anhydride);
a poly(acrylic acid);
a copoly(acrylic acid-acrylamide);
a polymer having a plurality of reactive groups and a plurality of ionizable groups, and at least one of the reactive groups has a spacer unit situated between the polymer backbone and the reactive group; and
a carboxy modified cellulose polymer;
and like polymers, a salt thereof, or a combination thereof.

In embodiments, the carboxy polymer can comprise, for example, mers of the formula:

**-{-CH(C(=O)(OH))-CH(C(=O)NH-R ²_(X))-R¹-}ₘ-**

where
R¹ is a divalent -(C₂₋₆ alkylene)-;
R² is a divalent spacer comprising from about 6 to about 30 atoms;
X is a reactive group or a salt thereof; and
m is from about 10 to about 10,000,
or a salt thereof, as disclosed for example, in commonly assigned copending application USSN 11/448,486, filed June 7, 2006, Patent Application Publication US 2007/0154348.

In embodiments, the carboxy polymer can be, for example, a copolymer comprised of mers of the formula:

**-{-CH(C(=O)(OH))-CH(C(=O)NH-R²-(X))-R¹-}ₘ-**

**-{-CH(C(=O)(OH))-CH(C(=O)-(Y))-CH-R¹-}ₙ-**

**-{-CH(C(=O)(OH))-CH(C(=O)NH-R³)-R¹-}₀-**

where
R¹ is a divalent -(C₂₋₆ alkylene)-;
R² is a divalent spacer comprising from about 6 to about 30 atoms;
R³ is a monovalent -(C₁₋₆ alkyl);
X is a reactive group or a salt thereof;
Y is a surface substantive group or a salt thereof;
m is from about 10 to about 10,000;
n is from about 10 to about 10,000; and
o is from about 10 to about 10,000,
or a salt thereof.

In embodiments, the mers of a specific spacer can be, for example:
R¹ is a -(-CH₂-CH₂-)-;
R² is a divalent spacer comprising a poly alkylene glycol segment containing from about 2 to about 6 alkylene glycol units;
R³ is a propyl group;
X is a sulfo-N-hydroxysuccinimide group or the salt thereof;
Y is a surface substantive group;
m is from about 10 to about 10,000;
n is from about 10 to about 10,000; and
o is from about 10 to about 10,000,
and a salt thereof.

In embodiments, a more specific spacer can have mers of, for example, an R² having a divalent spacer of the formula:

**-NR-(CH₂)₂-(O-CH₂-CH₂)ₚ-C(=O)-O-**

where each R is hydrogen or a monovalent -(C₁₋₆ alkyl), and p is from about 2 to about 6, for example, where p is nominally or exactly 4.

In embodiments,the linker and the protein can be in a relative mole ratio of, for example, from about 1:1 to about 50:1, from about 2:1 to about 25:1, and from about 5:1 to about 10:1, including intermediate values and ranges. The activator can be an activating agent selected from, for example, EDC, NHS, sNHS, DCC, DIC, HOBt, HBTU, TBTU, as mentioned above, and like agents, or a combination thereof.

In embodiments, the linker can be, for example, an amine of the formula H₂N-R¹-Y where R¹ is a branched or unbranched, substituted or unsubstituted (C₁₋₈) alkyl, or an oxyhydrocarbylene of the formula -CHR³-CHR³-(O-CHR³-CHR³)_{z}-, and Y is -H, - OH, -NH₂, -NHR², -NR₂², or a combination thereof, where R² is a branched or unbranched, substituted or unsubstituted (C₁₋₈)alkyl, or an oxyhydrocarbylene of the formula -CHR³-CHR³-(O-CHR³-CHR³)_{z}-, z is an integer of from 1 to about 10, and R³ can be H, or a branched or unbranched, substituted or unsubstituted (C₁₋₈)alkyl. An example of an oxyhydrocarbylene of the formula -CHR³-CHR³-(O-CHR³-CHR³)_{z}-, can be, for example, an oligomeric or polymeric alkylene oxide, such as a polyethylene glycol (PEG), a polypropylene glycol (PPG), or like oxyhydrocarbylenes. Numerous suitable compounds of the formula H₂N-R¹-Y as defined herein are commercially available, such as from Sigma-Aldrich.

In embodiments, the low pI protein for immobilization and detection can be present in an amount of, for example, from about 10 micrograms/mL to about 100 micrograms/mL, and the low pI protein can have a pI of, for example, from about 2 to about 6, and the linker-modified protein can be entrapped on the substrate in an amount, for example, having an indication from about 100 to about 10,000 picometers (pm or dimensionless relative units), from about 200 to about 7,500 pm, from about 400 to about 5,000 pm, from about 500 to about 2,500 pm, and from about 500 to about 1,000 picometers (pm), as measured by, for example, a resonant wave guide Epic^{®} instrument, including intermediate values and ranges. The linker-modified protein can have a modified isoelectric value (pI' ) of, for example, from about 4 to about 8, from about 4 to about 7, and from about 5 to about 7, including intermediate values and ranges.

In embodiments,the immobilized protein can be associated with the carboxy polymer coated surface by, for example, covalent, ionic, hydrophobic, or a combination of these and other associations.

In embodiments, the disclosure also provides articles having an entrapped protein thereon, articles having an immobilized protein thereon, or both, and to methods of using the articles having an entrapped or immobilized protein on the article's surface, such as in biosensing application, and like applications.

In embodiments, the disclosure provides supports useful for performing assays. In embodiments, a support for performing an assay comprises a substrate having a polymer directly or indirectly attached to the substrate, the polymer has a plurality of reactive groups that are capable of attaching-to or binding-with a biomolecule and a plurality of ionizable groups, the ratio of reactive groups to ionizable groups can be, for example, from about 0.5 to about 10, and the polymer can have a spacer unit or spacer groups situated between the polymer backbone and at least some of the reactive groups. In instances where the support is used for, for example, label independent detection methods, the polymer need not contain a photoactive group.

In embodiments, the disclosure provides an article for label-independent detection, the article comprising:
a substrate comprising a biomolecule-reactive contact surface; and
optionally a tie layer having the abovementioned biomolecule-reactive contact surface composition deposited on the tie layer.

The substrate can further include one or more biomolecules attached to any of the biomolecule immobilization groups or reactive groups.

In embodiments, the disclosure provides an article for label independent detection, the article comprising:
a substrate having a polymer coated contact surface comprising:
   a tie layer; and
   a polymer coat such as disclosed herein; and
   optionally a biomolecule attached to the contact surface.

In embodiments the disclosure provides an apparatus for label independent detection, the apparatus comprising: an optical biosensor having the aforementioned article having a polymer coated contact surface.

In embodiments, the disclosure provides an apparatus for label-independent detection, the apparatus comprising: an optical biosensor having a biomolecule-reactive contact surface comprising the abovementioned contact surface and optionally one or more biomolecules attached to or reacted with any of the biomolecule immobilization groups.

In embodiments, the disclosure provides methods for making a support article, comprising:
attaching a polymer directly or indirectly to a substrate, the polymer having a plurality of reactive groups capable of attaching or binding to a biomolecule and a plurality of ionizable groups, the ratio of reactive groups to ionizable groups can be, for example, from about 0.5 to about 10.0, and the polymer can have, for example, a plurality of spacer groups, each spacer group being situated between the polymer backbone and at least some of the reactive groups.

In embodiments the disclosure provides methods for making a support article, comprising:
attaching a polymer directly or indirectly to a substrate, the polymer having a plurality of biomolecule-reactive groups;
contacting the attached polymer with a spacer group reactant; and
contacting the resulting attached polymer having a spacer group with a biomolecule-reactive group reactant. The biomolecule-reactive group reactant can react with the spacer group to add a biomolecule-reactive site. The biomolecule-reactive group reactant can also react with other available groups on the polymer to add additional biomolecule-reactive sites thereto, if desired.

Suitable substrates can include, for example, a microplate, a slide, or any other material that is capable of attaching to the polymer. In embodiments, when the substrate is a microplate, the number of wells and well volume may vary depending upon, for example, the scale and scope of the analysis. Other examples of useful substrates can include, for example, a cell culture surface such as a 384-well microplate, a 96-well microplate, 24-well dish, 8-well dish, 10 cm dish, a T75 flask, or like articles.

For optical or electrical detection applications, the substrate can be, for example, transparent, impermeable, or reflecting, and electrically conducting, semiconducting, or insulating. For biological applications, the substrate material can be, for example, either porous or nonporous, and can be selected, for example, from organic or inorganic materials, or a combination thereof.

In embodiments, the substrate can be, for example, a plastic, a polymeric or copolymeric substance, a ceramic, a glass, a metal, a crystalline material, a noble or semi-noble metal, a metallic or non-metallic oxide, an inorganic oxide, an inorganic nitride, a transition metal, and like materials, or any combination thereof. Additionally, the substrate can be configured so that it can be placed in any detection device. In one aspect, sensors can be integrated into, for example, the bottom or underside of the substrate and used for subsequent detection. These sensors can include, for example, optical gratings, prisms, electrodes, quartz crystal microbalances, and like articles. Detection methods can include, for example, fluorescence, phosphorescence, chemiluminescence, refractive index, mass, electrochemical, and like detection methods. In embodiments, the substrate can be a resonant waveguide grating sensor.

In embodiments, the substrate can include an inorganic material. Examples of inorganic substrate materials can include, for example, metals, semiconductor materials, glass, ceramic materials, and like materials. Examples of metals that can be used as substrate materials include, for example, gold, platinum, nickel, palladium, aluminum, chromium, steel, gallium arsenide, or combination thereof. Semiconductor materials used for the substrate material can include, for example, silicon and germanium. Glass and ceramic materials used for the substrate material can include, for example, quartz, glass, porcelain, alkaline earth aluminoborosilicate glass and other mixed oxides. Further examples of inorganic substrate materials can include, for example, graphite, zinc selenide, mica, silica, lithium niobate, and inorganic single crystal materials. In embodiments, the substrate can be gold or gold coated, for example, a gold sensor chip.

In embodiments, the ratio of biomolecule reactive groups, such as NHS or sNHS, to ionizable groups, such as carboxyl -C(=O)(OH) groups, can be, for example, from about 0.01 to about 100 (0.01 ≤ R/I ≤ 100, where R/I represents the ratio of reactive(R) to ionizable groups (I)), and from about 0.1 to about 10.

When the number of reactive groups in the polymer is, for example, less than about 1% of all possible reactive and ionizable sites, the attachment of the biomolecules, while still possible will be less than highly efficient. Additionally or alternatively, if there is less than about 1% ionizable groups available of all possible reactive and ionizable sites, the attachment of the biomolecules and subsequent binding to the bound biomolecules will be less than highly efficient compared to when higher amounts of reactive and ionizable groups are present.

In embodiments, the ratio of reactive groups to ionizable groups can be, for example, from about 0.5 to about 5.0. In embodiments, the lower end of the ratio can be, for example, 0.5, 0.6, 0.7, 0.8, 0.9, 1.0, 1.5, 2.0, 2.5, 3.0, 3.5, 4.0, 4.5, or 5.0 and the upper end can be, for example, 2.0, 2.5, 3.0, 3.5, 4.0, 4.5, 5.0, 5.5, 6.0, 6.5, 7.0, 7.5, 8.0, 8.5, 9.0, 9.5, or 10.0, where any lower and upper end can form the ratio range. In embodiments, the ratio of reactive groups to ionizable groups can be, for example, from about 0.5 to 9.0, about 0.5 to 8.0, about 0.5 to 7.0, about 0.5 to 6.0, about 0.5 to 5.0, about 0.5 to 4.0, about 0.5 to 3.0, about 0.6 to 3.0, about 0.65 to 3.0, or about 0.67 to about 3.0.

A reactive group, such as an NHS, can also have an ionizable group, such as a sulfonic acid group found in for example a sulfo-NHS. If complete activation were possible, for example with a sulfo-NHS reagent or a reactive group former, the ratio of sulfonic acid groups per NHS group would be 1, that is, 1 sulfonic acid group per NHS reactive formed. However, activation is typically not complete or is less than 100%, so that the unreacted carboxylic acid groups make the ratio of biomolecule reactive groups to ionizable groups less than 1. The formation and number of reactive groups and ionizable groups present on the polymer can be controlled in a number of ways. In embodiments, the polymer can be synthesized from monomers possessing reactive groups and monomers with ionizable groups. In embodiments, the stoichiometry of the monomers selected can control the ratio of reactive groups to ionizable groups. In embodiments, a polymer possessing just reactive groups can be treated so that some of the reactive groups are converted to ionizable groups prior to attaching the polymer to the substrate. The starting polymer can be commercially available or synthesized using known techniques. In embodiments, a polymer can be attached to the substrate, and the attached polymer can be treated with various reagents to add either or both reactive groups and ionizable groups, or convert reactive groups to ionizable groups. In embodiments, a polymer that possesses reactive groups can be attached to the substrate, where the substrate reacts with the reactive groups and produces ionizable groups.

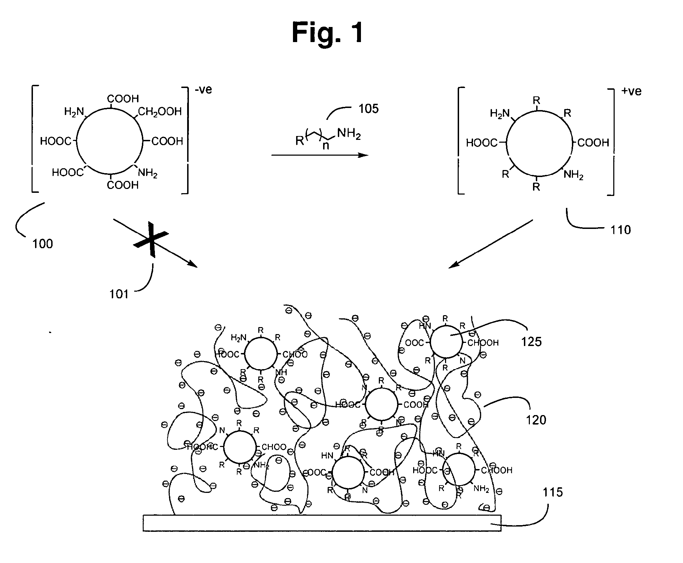

Referring to the Figures, Fig. 1 schematically shows an exemplary chemical cationization of a low pI protein and its subsequent entrapment and immobilization with a negatively biased surface. A low pI protein (**100**) having, for example, ionizable carboxylic acid and amine groups, or like groups, will typically be repelled or blocked (**101**) from a negatively biased surface, and will not productively associate with such a surface. In embodiments of the method of the disclosure, the solution charge properties (-ve) of a low pI protein (e.g., **100**) can be chemically modified, for example, with a linker compound, such as an organic amine compound (**105**), to afford a structurally modified protein (**110**) having modified charge properties (+ve), that is, an elevated pI and a positive charge at a pH used for the immobilization. The structurally modified protein (**110**) having, for example, organic amine R group substituents replacing or protecting former carboxy groups, now has a greater opposite charge, greater charge attraction, and less charge repulsion with respect to a negatively biased surface, such as an ionized polymer coated substrate **(115+120).** Structurally modified protein **(110)** can be entrapped-in (**125**) the carboxy polymer surface modified substrate **(115/120),** and can be subsequently immobilized with the modified substrate **(115/120)** in significantly greater amounts compared to the unmodified protein to permit a meaningful detection and analysis of the immobilized entity.

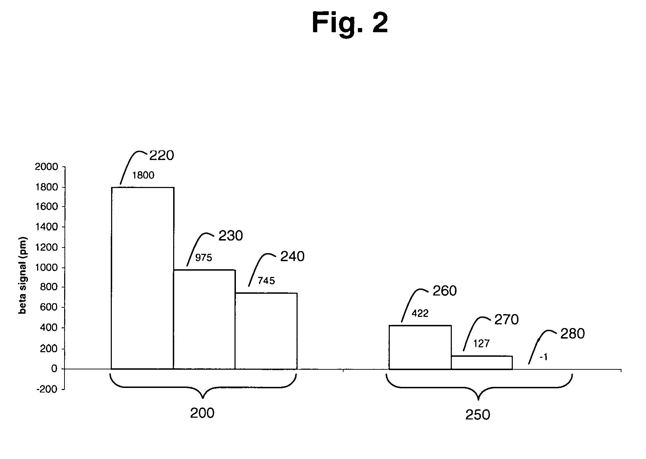

Fig. 2 shows exemplary cationized low pI proteins **(200)** and comparative unmodified low pI proteins **(250)** (control) immobilization results. Both the cationized and unmodified proteins were in an acetate buffer (20 mM, pH 5.5). Cationized low pI proteins, where pI' is the cationized pI value, included: amine-modified or amine-coupled trypsin inhibitor (pI' = 5 to 7)**(220)**, calmodulin (pI' = 5 to 7) **(230),** and amyloglycosidase (pI' = 5 to 6) **(240)** and were compared with the corresponding chemically unmodified or uncationized native proteins: trypsin inhibitor (pI = 4.6) **(260),** calmodulin (pI = 4.0) **(270),** and amyloglycosidase (pI = 3.6) **(280).** The measured values (in picometer relative units), is indicated by the respective cardinal numbers atop the reference number labeled columns. The pI of the modified protein is not an exact number or a single value. Instead a range or distribution of pI values for individual proteins provides an average pI over the ensemble since differing levels of modification yield per protein are expected.

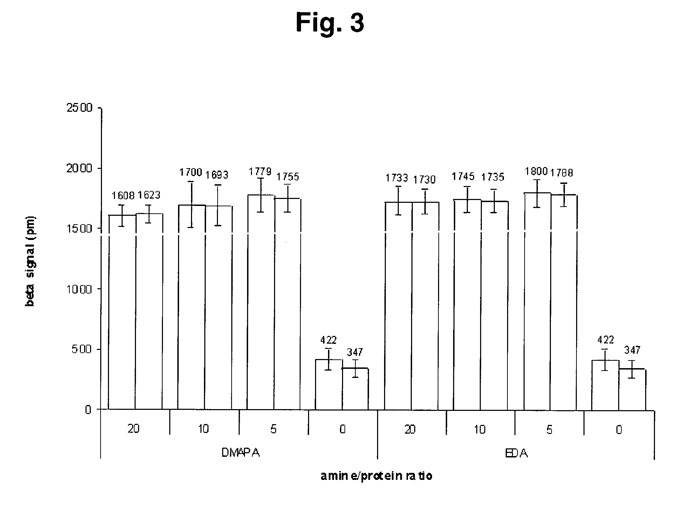

Fig. 3 shows the immobilization levels for different linker to protein mole ratios (20:1, 10:1, 5:1, and 0:0 linker:protein mole ratio) where the same low pI protein, trypsin inhibitor (pI 4.6; 21.5 kDa immobilized at 100 microgram/mL in acetate buffer on propyl amine derivatized EMA), was selected for linker modification. The linker was either 3-(dimethylamino)-1-propylamine (DMAPA) or ethylenediamine (EDA). Linker modification was accomplished in accordance with Example 1. The level of immobilized modified protein is indicated by the magnitude of the beta signal for each of the linkers before (left-hand column) and after (right-hand column) plate washing. The measured values, in picometer relative units, are indicated by the respective cardinal numbers in the body of the reference-number labeled-columns.

Table 3 lists the immobilization results of Example 3 for different linker/protein ratios where the protein was trypsin inhibitor and the linker was either 3-(dimethylamino)-1-propylamine amine (DMAPA) or ethylenediamine (EDA). The before- and after-plate washing values indicated that the ratio of entrapment to immobilization was near unity. Immobilized protein level (pm) refers to the level or amount of protein that was immobilized as measured by an Epic^{®} biosensor in relative units of picometers, and Immob. SD(+) refers to the immobilization standard deviation in the positive wells.

**Table 3.**

| | | **BEFORE WASH** | | **AFTER WASH** | |
|---|---|---|---|---|---|
| **Linker** | **Linker-protein mole ratio** | **Immobilized protein level (pm)** | **Immob. SD(+)** | **Immobilized protein level (pm)** | **Immob. SD(+)** |
| **DMAPA** | 20 | 1608 | 89 | 1623 | 74 |
| | 10 | 1700 | 190 | 1693 | 167 |
| | 5 | 1779 | 141 | 1755 | 118 |
| | 0 | 422 | 88 | 347 | 78 |
| **EDA** | 20 | 1733 | 121 | 1730 | 103 |
| | 10 | 1745 | 108 | 1735 | 95 |
| | 5 | 1800 | 117 | 1788 | 97 |
| | 0 | 422 | 88 | 347 | 78 |

The results shown in the Table 3 and Fig. 3 show the difference in the Epic^{®} biosensor signal for a low pI modified protein and its unmodified protein. It can be seen that the unmodified protein does not readily bind to the dEMA matrix so that the Epic^{®} signal is very low, at roughly 400 pm. This typically corresponds to the expected level of non-specific binding of a protein to a surface. Upon modification with either 20, 10, or 5 mole equivalents of DMAPA or EDA the immobilization level rose dramatically to about 1,700 to about 1,800 pm. In this experiment there was little difference between the different linkers, or the amount of linker used. The amount of linker used can be optimized, if desired, for each particular protein. A sufficient amount of linker should be added to raise the pI to a minimum value that allows preconcentration to take place. However, care must be exercised as unfavorable effects can be expected when modification of the protein's carboxyl groups are too extensive and can impair the protein's function and stability (see Futami, et al., *supra*).

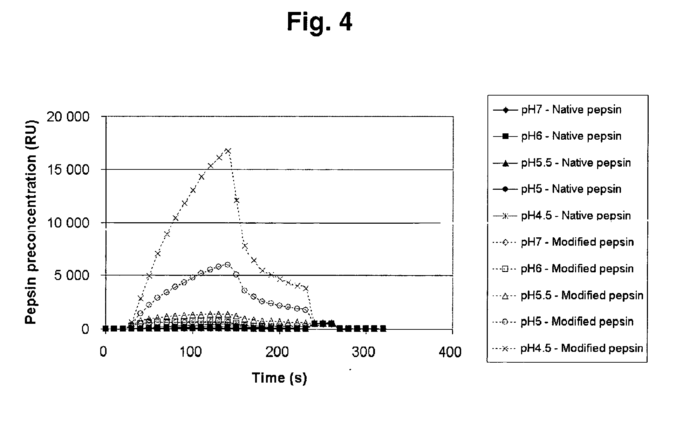

Fig. 4 is a sensorgram that shows representative levels of entrapped protein over time with injection and removal with the running buffer for the respective cationized(+) and the native(N) proteins at a pH between about 4.5 and about 7. The amount of native or cationized trypsin inhibitor protein entrapped over time is in relative units (RU) as measured with an Epic^{®} instrument. High signal response traces (400), (410), (420), (430), and (440) correspond to the cationized proteins, whereas low signal response traces (450), (460), (470), (480), and (490) correspond to the native proteins.

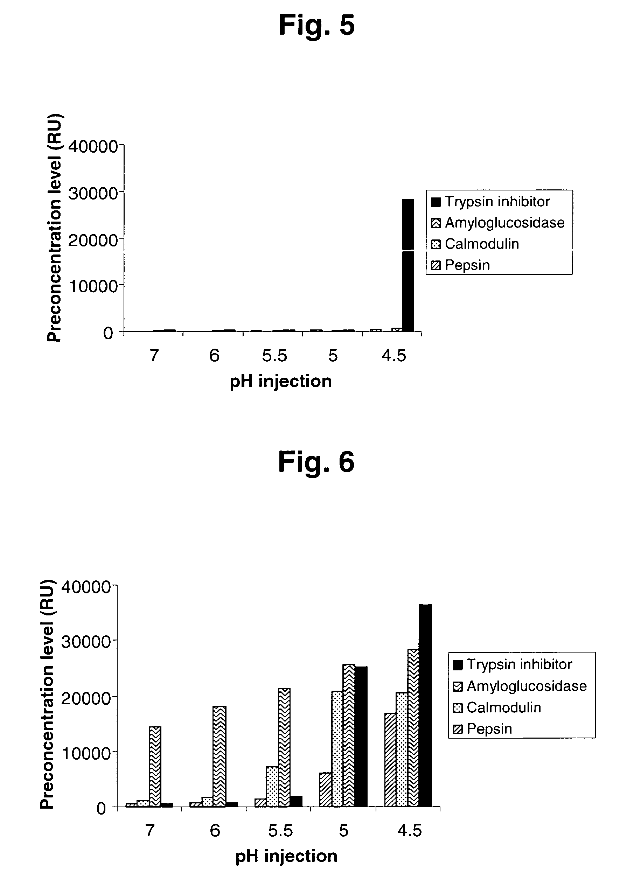

Figs. 5 and 6 show the amount of protein, native and cationized, respectively, entrapped in the CM5 surface layer at different pH levels.

### EXAMPLES

The following examples serve to more fully describe the manner of using the above-described disclosure, as well as to set forth the best modes contemplated for carrying out various aspects of the disclosure. It is understood that these examples do not limit the scope of this disclosure, but rather are presented for illustrative purposes.

The working examples further describe how to make and use the articles and methods of the disclosure.

Isoelectric focusing ("IEF") gel methods can be used, for example, to quantify a native or target protein's pI, and the protein's pI and change in pI in upon, for example, linker derivatization in accordance with the disclosure.

The starting materials, such as low pI proteins, and like materials, are commercially available, such as from Sigma-Aldrich an like suppliers; can be, for example, prepared by known methods; can be isolated from a complex matrix by known methods, and like sources and methods. All commercially available chemicals were used as received.

### Materials

Trypsin inhibitor from Glycine max (soybean) (Sigma)(21.5 kDa, pI 4.6). 3-(dimethylamino)-1-propylamine (DMAPA) (Aldrich).
Ethylenediamine (EDA) (Aldrich).
Acetate buffer (Fisher, 0.410 g NaOAc in 250 mL H₂O adjusted to pH 5.5 using 1M HCl).
N-(3-dimethylaminopropyl)-N'-ethylcarbodiimide hydrochloride (EDC) (Pierce).

All reactions and processes, including chemical derivatizing, entrapping, immobilizing, and subsequent interactions with a ligand, were accomplished at a pH approximating or matching the pH for the protein's native environment. The pH was never below 1 or above 13. All reactions were accomplished at room temperature (ca. 25 °C) and proteins, or modified proteins, were stored at -20°C.

### Example 1

**Preparation of linker-modified low pI protein.** Linker solutions were prepared containing 1.2x10⁻², 6x10⁻³ and 3x10⁻³ mmol/mL of either DMAPA or EDA linker compound. To 130 microliters protein solution in water (1 mg/mL) was added 20 microliters 0.4 M EDC and 10 microliters of linker solution. These mixtures were allowed to react for approximately 1.5 hr and diluted 1:9 with acetate buffer to give final protein concentration of about 100 micrograms/mL.

### Example 2

**Entrapping linker-modified protein on a derivatized EMA substrate surface.** 15 microliters of each solution was added to the wells of a derivatized EMA Epic^{®} well plate commercially available from Coming, Inc., and a 10 microliter mixing cycle was applied. The plate was centrifuged at 800 rpm for 1 min and allowed to stand for 1 hr. The plate was thoroughly rinsed with 25 microliter of phosphate buffer solution (PBS) (Sigma P4417; 1 tablet dissolved in 200 mL of ultra-pure water and the solution filtered five times (pH 7.5). Finally, 15 microliters of PBS was added to each well and the plate was centrifuged at 800 rpm for 1 min. The plate was read before and after the rinsing step in an Epic^{®} biosensor instrument adapted from temperature control and sample handling. The results are presented graphically in Fig. 3.

### Example 3

### Entrapping linker-modified protein on a polysaccharide surface; Method and results on the Biacore^{®} T100 instrument

Proteins used:
Pepsin hog stomach (Pepsin A), 41.3 kDa, pI 4.0
Calmodulin, from bovine testes, 16.3 kDa, pI 4.0
Amyloglucosidase from *aspefillus niger,* 97 kDa, pI 3.6
Trypsin inhibitor from Glycine max, 21.5 kDa, pI 4.6

Other chemicals:
Ethylenediamine (EDA) (Aldrich) 11 microliters in 1 ml diluted 1/10 twice.
Acetate buffer (Fisher), 0.410 g NaOAc in 250 ml H₂O adjusted to pH 4.45, 4.95, 5.5, 6.05 and 6.8 using 1M HCl.
N-(3-Dimethylaminopropyl)-N'-ethylcarbodiimide hydrochloride (EDC), (Pierce) @ 0.4 mM.

Protein Preparation:
To a solution of protein at 1 mg/mL(100 microliters) was added 14 microliters of dilute ethylenediamine (EDA) solution and 14 microliters EDC solution. The ratio of protein to linker was approximately:
   1:10 for Pepsin A
   1:4 for Calmodulin
   1:23 for Amyloglucosidase
   1:5 for Trypsin inhibitor
The proteins were allowed to react for 1.5 h and stored at -20 °C.

Biacore^{®} pH scouting
Running buffer = acetate buffer 20 mM, pH 5.5
Injection buffer = acetate buffer 20 mM, pH = 7, 6, 5.5, 5, and 4.5
The procedure was carried out on Biacore^{®}'s CM5 proprietary surface. Proteins were diluted in injection buffer at 100 micrograms/mL and injected for 120 seconds at 10 microliters/minute. The channel was then washed with running buffer followed by an injection of 50 mM NaOH to remove all adsorbed protein and regenerate the surface. The procedure was repeated for the five different injection buffers beginning with pH 7 and finishing at pH 4.5.

### Results

For the CM5 surface none of the native proteins showed entrapment except for the trypsin inhibitor at pH 4.5. This was expected as the pI of this protein is 4.6, so that at pH 4.5 the entrapment was accomplished at a pH below the pI of the protein.

The modified proteins show very different results. All cationized proteins were entrapped at high levels at pH 4.5, between about 15,000 and about 40,000 response units (RU). All proteins, except pepsin A still showed entrapment levels between about 20,000 and about 25,000 RU at pH 5. After that the entrapment levels drop steadily. Amyloglucosidase showed the highest entrapment level even at pH 7 with 14,000 RU. The amount of linker added to the amyloglucosidase was higher due to its higher molecular mass.

### Example 4

### Entrapping linker-modified Pepsin A protein on modified surfaces; Method and results on the Epic^{®} instrument

Protein: Pepsin hog stomach (Pepsin A), 41.3 kDa, pI 4.0
Other chemicals:
   Ethylenediamine (EDA) (Aldrich) 11 microliter in 1 mL diluted 1/10 twice.
   Acetate buffer (Fisher, 0.410 g NaOAc in 250 mL H₂O adjusted to pH 4.0 4.5, 5.0 and 5.5 1M HCl.
   N-(3-Dimethylaminopropyl)-N'-ethylcarbodiimide hydrochloride (EDC), (Pierce) at 0.4 mM.
   Pepsin A was cationized with 10 equivalent of ethylene diamine prior to contacting with selected surfaces dEMA, spacer, and PAA/DMAm as defined herein, and detecting with an Epic^{®} instrument.
   A solution of EDA (11 microliters) in water (989 microliters) was diluted 1/10 twice. To 880 microliters of pepsin in water (1 mg/mL) was added 126 microliters dilute EDA solution and 126 microliters EDC solution (0.4 mM) and the reaction was allowed to stand for 1.5 h. The resultant solution was diluted to a final protein concentration of 100 microg/mL using acetate buffer at pH 4.0, 4.5, 5.0, or 5.5. Next, 15 microliters of each solution was added to the wells of each of following plates:
      "dEMA" in this example refers to a propyl amine derivatized ethylene-maleic anhydride (dEMA) Epic^{®} plate, which plate is commercially available from Coming, Inc.;
      "Spacer" refers to a spacer modified dEMA polymer on an Epic^{®} plate and as disclosed herein and in copending provisional patent application USSN 61/123609, filed April 10, 2008, entitled "SURFACE FOR LABEL INDEPENDENT DETECTION AND METHOD THEREOF." Specifically, the spacer polymer can comprise, for example, mers of the abovementioned formula:

         -{-CH(C(=O)(OH))-CH(C(=O)NH-R²-(X))-R¹-}ₘ-

         -{-CH(C(=O)(OH))-CH(C(=O)-(Y))-CH-R¹-}ₙ-

         -{-CH(C(=O)(OH))-CH(C(=O)NH-R³)-R¹-}ₒ-
      where
      R¹ can be a -(-CH₂-CH₂-)-;
      R² can be a divalent spacer of the formula:

         -NR-(CH₂)₂-(O-CH₂-CH₂)ₚ-C(=O)-O-

         where each R can be hydrogen or a monovalent -(C₁₋₆ alkyl);
      R³ is a propyl group;
      X is a sulfo-N-hydroxysuccinimide group or the salt thereof;
      Y is a surface substantive group or a salt thereof;
      m is from about 10 to about 10,000;
      n is from about 10 to about 10,000;
      o is from about 10 to about 10,000; and
      p can be from about 2 to about 6, see for example Table 3 where p is 4,
and a salt thereof.

"PAA/DMAm 50/50" refers to an polyacrylic acid and dimethylacrylamide copolymer (1:1=mole ratio) coated on an Epic^{®} plate and chemically activated as disclosed in copending patent application European Application No. 08305845.3, filed Nov. 26, 2008. The PAA/DMAm 50/50 plate was prepared as follows: 2 wt % methacryloxypropyltrimethoxysilane (MOPS) (Sigma-Aldrich) aqueous solution containing acetic acid to give a pH of about 3.5 to about 4 was prepared and stirred until clear (e.g., 15 min at about 22°C). An uncoated Epic^{®} insert was used as received and without an additional cleaning step. The insert was contacted with the MOPS solution for 30 mins then dried under argon flow and dried at 50°C for 1 hours to ensure condensation of the silane with the insert surface. Then the insert was cooled to ambient temperature of about 25°C, rinsed with DI water, then with IPA, and finally dried with argon flow. The MOPS treated insert was contacted with about a 5 to 10 micron thick layer of an aqueous monomer mixture of dimethylacrylamide (50.3 mole %), acrylic acid (49.6 mole %) and Irgacure^{™} 184 (Ciba) mole %. 20 mg of Irgacure^{™} 184 were weighted in a flask and 7 ml of DI water added. Then 1.25 g of acrylic acid and 1.75 g of dimethylacrylamide was added. The flask was sealed with a septum and the solution was degassed with bubbling argon for about 15 min. Next, 500 microliters of the degassed monomer mixture was deposited onto the silane treated insert surface. The thickness of the monomer aqueous solution was controlled by clamping together with a hydrophobic treated glass cover plate (FDS fluorosilane). In-situ polymerization was performed with UV light using a fusion lamp (bulb H, 80% power, 1 pass at the minimum belt speed). Then the cover plate was removed and the adhering gel layer was thoroughly rinsed with DI water in an ultrasonic bath in order to remove excess material and the surface was finally dried under a gentle argon flow. Following polymerization and cleaning, the Epic^{®} insert was combined with a Coming Inc., holey well-plate using double-sided pressure sensitive adhesive (PSA) adhesive tape. The carboxylic acid groups of the polymer matrix were activated (using a Biomek automation workstation) by treatment with 5 mM sulfo-NHS and 20 mM EDC dissolved in water. At this stage, the activated polymer surface may be reacted with proteins, or other biomolecule that can be immobilized by amine coupling. A 10 microliter mixing cycle was applied and the plate was centrifuged at 800 rpm (1 min) and allowed to stand for about 1 h at about 25 °C and then 16 h at 4 °C. The plate was thoroughly rinsed with acetate buffer pH 5.5. Finally 15 microliters of acetate buffer pH 5.5 was added to each well and the plate was centrifuged at 800 rpm (1 min). The plate was read in the Epic^{®} Beta instrument.

### Results

The native pepsin A protein did not immobilize on any of the tested surfaces, in any significant amount, at any of the pH level tested. The Epic^{®} signal was between about 300 and about 1,000 pm.

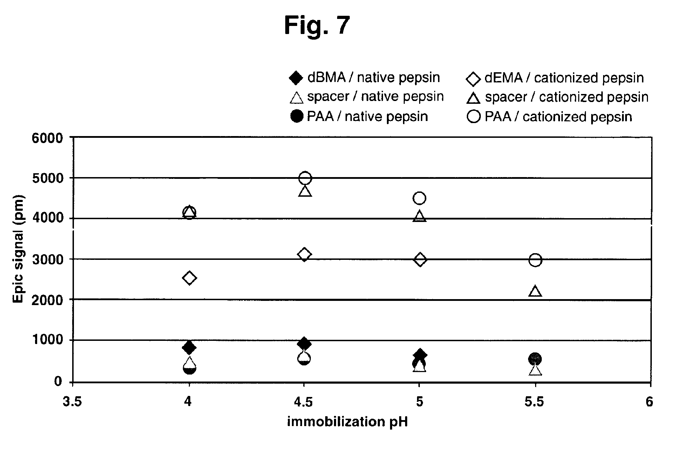

In contrast, the cationized pepsin A protein was immobilized at levels as high as about 2,500 to about 3,500 pm for the dEMA, and about 4,000 to about 5,000 pm for the dEMA-spacer or the PAA/DMAm surfaces. It is difficult to observe a small molecule binding to proteins immobilized at less than about 1,000 pm. However, between about 2,500 and about 5,000 pm a good signal for a small molecule binding event can be expected. The results are listed in Table 4. Fig. 7 graphically shows the pepsin A protein immobilization results for native and cationized proteins with selected surfaces using an Epic^{®} instrument.

**Table 4.**

| **Surface Polymer** | **acetate pH** | **protein** | **Protein Immob. level (pm)** | **Immob. SD(+)** |
|---|---|---|---|---|
| **dEMA** | pH 4 | native | 831 | 148 |
| | | cationized | 2,529 | 106 |
| | pH 4.5 | native | 905 | 98 |
| | | cationized | 3,123 | 182 |
| | pH 5 | native | 627 | 112 |
| | | cationized | 2,991 | 259 |
| **spacer** | pH 4 | native | 468 | 373 |
| | | cationized | 4,210 | 467 |
| | pH 4.5 | native | 668 | 300 |
| | | cationized | 4,722 | 399 |
| | pH 5 | native | 375 | 327 |
| | | cationized | 4,071 | 448 |
| | pH 5.5 | native | 353 | 329 |
| | | cationized | 2,237 | 453 |
| **PAA/DMAm** | pH 4 | native | 305 | 326 |
| | | cationized | 4,135 | 548 |
| | pH 4.5 | native | 550 | 407 |
| | | cationized | 4,993 | 476 |
| | pH 5 | native | 403 | 276 |
| | | cationized | 4,491 | 406 |
| | pH 5.5 | native | 546 | 282 |
| | | cationized | 2,979 | 402 |

The disclosure has been described with reference to various specific embodiments and techniques. However, it should be understood that many variations and modifications are possible while remaining within the spirit and scope of the disclosure.

## Claims

1. A method to entrap a protein, comprising:
contacting the protein and a mixture comprised of an activator, a linker, and a buffer solution, to form a linker-modified protein; and
contacting the linker-modified protein and a substrate to entrap the linker-modified protein on the substrate,
the substrate comprises a buffer solution swollen carboxy polymer coated surface having a pH, and the isoelectric point (pI) of the protein prior to linker modification is less than the pH of the buffer solution used to swell the carboxy polymer surface.

2. The method of claim 1, wherein the carboxy polymer surface comprises at least one of:
an alkyl-amine modified poly(alkylene-alt-maleic anhydride);
a poly(acrylic acid);
a copoly(acrylic acid-acrylamide);
a polymer having a plurality of reactive groups and a plurality of ionizable groups, and at least one of the reactive groups has a spacer unit situated between the polymer backbone and the reactive group;
a carboxy modified cellulose polymer; and
a salt thereof, or any combination thereof.

3. The method of claim 1 or claim 2, wherein the pH of the buffer used to swell the polymer coated surface is from about 0.5 to about 6 and the protein comprises at least one protein having a pI of from about 2 to about 5.

4. The method according to any one of claims 1 to 3, wherein the carboxy polymer surface comprises a reaction product of a poly(alkylene-alt-maleic anhydride) and an alkyl-amine having from about 30 to about 50 mol% of the anhydride groups reacted with propylamine.

5. The method according to any one of claims 1 to 4, wherein the linker and the protein are in a relative mole ratio of from about 1:1 to about 50: 1.

6. The method according to any one of claims 1 to 5, wherein the activator comprises an activating agent comprising a reagent selected from EDC, NHS, sNHS, HOBt, BOP, HBTU, DCC, DIC, TBTU, or a combination thereof.

7. The method according to any one of claims 1 to 6, wherein the linker comprises an amine of the formula H₂N-R¹-Y where R¹ is a branched or unbranched, substituted or unsubstituted (C₁₋₈) alkyl, or an oxyhydrocarbylene glycol of the formula -CHR³-CHR³-(O-CHR³-CHR³)_{z}-, and Y is -H, -OH, -NH₂, -NHR², -NR₂², or a combination thereof, where R² is a branched or unbranched, substituted or unsubstituted (C₁₋₈)alkyl, or an oxyhydrocarbylene of the formula -CHR³-CHR³-(O-CHR³-CHR³)_{z}-, z is an integer of from 1 to about 10, and R³ is H, or a branched or unbranched, substituted or unsubstituted (C₁₋₈)alkyl.

8. The method according to any one of claims 1 to 7, wherein the linker comprises at least one amine selected from 3-(dimethylamino)-1-propylamine, ethylenediamine, ethanol amine, 2-(2-aminoethoxy)ethanol, 2,2'-oxybis(ethylamine), 1,8-diamino-3,6-dioxaoctane, or a combination thereof.

9. The method according to any one of claims 1 to 8, wherein the protein is present in an amount of from about 10 micrograms/mL to about 100 micrograms/mL, and the linker-modified protein is entrapped on the substrate at an indication of from about 400 to about 10,000 picometers as measured by a resonance wave guide biosensor.

10. The method according to any one of claims 1 to 9, further comprising forming at least one covalent bond between the substrate surface and the modified protein and rinsing the protein contacted substrate with buffer to remove free protein.

11. A method for detecting a protein, comprising:
modifying the charge on the protein by contacting the protein with a mixture of an activating agent and an amino-linker compound to form an amino-linker modified protein;
contacting a carboxy polymer surface and the amino-linker modified protein to immobilize the amino-linker modified protein on the surface; and
detecting the immobilized amino-linker modified protein with a biosensor,
the carboxy polymer surface comprises a buffer swollen surface having a pH from about 0.5 to about 6, and the isoelectric point (pI) of the protein being less than the pH of the substrate.

12. The method of claim 11, wherein the linker and the protein are in a relative mole ratio of from about 1:1 to about 50:1, the activator comprises an activating agent selected from EDC, NHS, sNHS, HOBt, BOP, HBTU, DCC, DIC, TBTU, or a combination thereof, and the linker comprises an amine of the formula H₂N-R¹-Y where R¹ is a branched or unbranched, substituted or unsubstituted (C₁₋₈) alkyl, or an oxyhydrocarbylene glycol of the formula - CHR³-CHR³-(O-CHR³-CHR³)_{z}-, and Y is -H, -OH, -NH₂, -NHR², -NR₂², or a combination thereof, where R² is a branched or unbranched, substituted or unsubstituted (C₁₋₈)alkyl, or an oxyhydrocarbylene of the formula -CHR³-CHR³-(O-CHR³-CHR³)_{z}-, is an integer of from 1 to about 10, and R³ is H, or is a branched or unbranched, substituted or unsubstituted (C₁₋₈)alkyl.

13. The method of claim 11 or claim 12, wherein the protein is in an amount of from about 10 to about 100 micrograms/mL and has a pI of from about 2 to about 5, and the linker-modified protein is entrapped on the surface to an extent of about 400 to about 5,000 picometers as measured by a resonance wave guide biosensor.

14. The method according to any one of claims 1 to 13 further comprising contacting the immobilized linker-modified protein on the substrate with ligand.

15. An article as may be obtained by the method according to any one of claims 1 to 10.
